# EUROPEAN PATENT SPECIFICATION

(11) **EP 3 311 166 B1**
(45) Date of publication and mention of the grant of the patent: **29.04.2020**
(21) Application number: 16736007.2
(22) Date of filing: 17.06.2016
(51) Int. Cl.: G01N 33/574

(54) **CANCER DIAGNOSTIC**
KREBSDIAGNOSE
DIAGNOSTIC DU CANCER

(30) Priority: 19.06.2015 GB 201510880
(43) Date of publication of application: 25.04.2018
(73) Proprietor: University Of Kent, School Of Pharmacy, Chatham, Kent ME4 4TB (GB)
(72) Inventor: SUMBAYEV, Vadim, Chatham Kent ME4 4TB (GB); USHKARYOV, Yuri, Chatham Kent ME4 4TB (GB); GIBBS, Bernhard, Chatham Kent ME4 4TB (GB)
(74) Representative: Boxall, Sarah Jane
(86) International application number: PCT/EP2016/064104
(87) International publication number: WO 2016/203031

(56) References cited:
- WO-A2-2013/169771
- WO-A2-2014/168922
- J.-P. SILVA ET AL: "Latrophilin 1 and its endogenous ligand Lasso/teneurin-2 form a high-affinity transsynaptic receptor pair with signaling capabilities", PROCEEDINGS OF THE NATIONAL ACADEMY OF SCIENCES, vol. 108, no. 29, 1 July 2011 (2011-07-01) , pages 12113-12118, XP055297331, US ISSN: 0027-8424, DOI: 10.1073/pnas.1019434108
- VADIM V SUMBAYEV ET AL: "Expression of functional neuronal receptor latrophilin 1 in human acute myeloid leukaemia cells", ONCOTARGET, vol. 7, no. 29, 14 June 2016 (2016-06-14), pages 45575-45583, XP055297121,

## Description

The present invention relates to the diagnosis of haematopoietic cell cancer using expression of latrophilin as a biomarker. Methods and kits for the detection and stimulation of Istrophilin are also disclosed.

Haematopoietic cells, haematopoietic stem cells (HSCs) or haemocytoblasts are the cells that give rise to all the other blood cells through the process of haematopoiesis. They are derived from mesoderm and located in the red bone marrow, which is contained in the core of most bones.

The cells give rise to both the myeloid and lymphoid lineages of blood cells. Myeloid cells include monocytes, macrophages, neutrophils, eosinophils, erythrocytes, dendritic cells, and megakaryocytes or platelets. Lymphoid cells include T cells, B cells, and natural killer cells.

Leukaemia is a group of cancers that usually starts in blood-forming tissue, including the bone marrow. It leads to the over-production of abnormal white blood cells, the part of the immune system that defends the body against infection.

Blood cells are formed in the bone marrow, the tissue found inside the bones. Blood-forming stem cells divide to produce either more stem cells or immature cells that become mature blood cells over time. A blood stem cell may become a "myeloid" stem cell or a "lymphoid" stem cell.

A myeloid stem cell becomes one of three types of mature blood cells:
- Red blood cells that carry oxygen and other substances to all tissues of the body.
- Platelets that form blood clots to stop bleeding.
- Myeloid leucocytes (a subtype of white blood cells) that fight pathogens.

A lymphoid stem cell becomes a lymphoblast cell and then one of three types of lymphocytes (another subtype of white blood cells):
- B lymphocytes that develop either into plasma cells (otherwise known as plasma B cells, plasmocytes or effector B cells), which make antibodies to help fight infection, or memory B cells.
- T lymphocytes that include cytotoxic T cells (which destroy infected cells or tumour cells), helper T cells (which either support the activation of B lymphocytes or activate cytotoxic T cells) and macrophages.
- Natural killer (NK) cells that attack cancer cells and cells infected by viruses.

Leukaemia affects white blood cells and may be classified either by the type of white cell affected (myeloid or lymphoid) or by the way the disease progresses (acute or chronic). Acute and chronic do not refer to how serious the disease is but to how rapidly it progresses.

Acute leukaemia is characterised by a rapid increase in the number of malignantly transformed immature blood cells. Crowding, due to the presence of such cells, makes the bone marrow unable to produce healthy blood cells. Immediate treatment is required due to the rapid progression and accumulation of the malignant cells, which then spill over into the bloodstream and spread to other organs of the body. Acute forms of leukaemia are the most common forms of leukaemia in children and may often be cured with standard treatments, such as bone marrow transplants, especially in younger and/or fitter patients.

Chronic leukaemia is characterised by the excessive build up of relatively mature, but still abnormal, malignantly transformed white blood cells. Typically taking months or years to progress, these cells are produced at a much higher rate than normal, resulting in many abnormal white blood cells. Whereas acute leukaemia must be treated immediately, chronic forms are sometimes monitored for some time before treatment to ensure maximum effectiveness of therapy. Chronic leukaemia mostly occurs in older people but may theoretically occur in any age group. Although it can be treated and managed, it is not usually possible to cure chronic leukaemia with standard treatments.

The acute and chronic diseases are also subdivided according to the type of blood cell affected. This divides leukaemias into i) lymphoblastic or lymphocytic leukaemias and ii) myeloid or myelogenous leukaemias.

In lymphoblastic or lymphocytic leukaemias, the cancerous change takes place in a type of bone marrow cell that normally goes on to form various types of lymphocytes. Most lymphocytic leukaemias involve a specific subtype of lymphocyte, the B cell.

In myeloid or myelogenous leukaemias, the cancerous change takes place in a type of immature cells that normally goes on to form red blood cells, some other types of white cells, and platelets.

Combining these two classifications provides a total of four main categories. Within each of these four main categories, there are typically several subcategories.
- Acute myeloid leukaemia (AML) occurs more commonly in adults than in children, and more commonly in men than women, is rapidly developing and affects myeloid cells. Subtypes of AML include acute promyelocytic leukaemia, acute myeloblastic leukaemia, and acute megakaryoblastic leukaemia.
- Chronic myeloid leukaemia (CML) occurs mainly in adults, develops slowly and affects myeloid cells. One subtype is chronic myelomonocytic leukaemia.
- Acute lymphoblastic leukaemia (ALL) is the most common type of leukaemia in young children, is rapidly developing and affects lymphocytes. Subtypes include precursor B acute lymphoblastic leukaemia, precursor T acute lymphoblastic leukaemia, Burkitt's lymphoma, and acute biphenotypic leukaemia.
- Chronic lymphocytic leukaemia (CLL) is slowly developing, affects lymphocytes and is most commonly seen in adults over the age of 55. One subtype is B-cell prolymphocytic leukaemia, a more aggressive disease.

Rarer types of leukaemia that are usually considered to be outside the above classification scheme include Hairy cell leukaemia (HCL), T-cell prolymphocytic leukaemia (T-PLL), Large granular lymphocytic leukaemia and Adult T-cell leukaemia.

Another type of leukaemia is mast cell leukaemia. This is an extremely aggressive subtype of acute myeloid leukemia that usually occurs *de novo* but can, rarely, evolve from transformation of chronic myeloid leukemia into the more aggressive acute myeloid leukemia. In a small proportion of cases, acute mast cell leukemia may evolve from a more progressive form of systemic mastocytosis. The diagnosis of acute mast cell leukemia by the World Health Organsiation (WHO) criteria includes the requirement for a prevalence of 20% neoplastic mast cells in marrow and 10% in blood. If the mast cells represent less than 10% of blood cells, the tumor is called "aleukemic" mast cell leukemia.

In some cases, mast cell leukaemia begins as mast cell sarcoma. This is an extremely aggressive form of sarcoma made up of neoplastic mast cells. Mast cell sarcoma is an extremely rare tumor and prognosis is extremely poor. People with a mast cell sarcoma have no skin lesions, and pathology examination of the tumor shows it to be very malignant with an aggressive growth pattern.

Diagnosis of haematopoietic cell cancers such as leukaemia is typically based on repeated blood tests and bone marrow biopsy that can only be assessed in a laboratory. The results of a simple blood count will usually indicate for example leukaemia although, sometimes, a blood count may be normal, especially in the early stages of the disease or during remission. Most patients with leukaemia will have a bone marrow sample taken to confirm the diagnosis and to help determine exactly what type of leukaemia a patient has. A lymph node biopsy may also be performed to diagnose certain types of leukaemia in certain situations.

Following diagnosis, blood chemistry tests are typically used to determine the degree of liver and kidney damage or the effects of chemotherapy on the patient. In some, but not all, types of haematopoietic cell cancers a sample is taken of the cerebrospinal fluid that surrounds the brain and spinal cord. This is because some types of haematopoietic cancerous cells are able to enter the nervous system, which protects them from most kinds of treatment.

Recent evidence demonstrates that malignant transformation of leucocytes leads to a strong increase in the expression of certain surface proteins, for example Tim-3 and CD123 (Prokhorov A. et al(2015) Int. J. Biochem. Cell Biol. 59 11-20; Tettamanti S. et al(2014) Oncoimmunology 3 e28835).

A significant drawback of currently known biomarkers for leukaemia is that the biomarkers are expressed on both healthy and malignant white blood cells and so diagnosis relies on accurate and repeatable measurement of up- or down-regulation of a marker. To improve biomarker specificity, co-expression of receptors is sometimes considered. For example, progenitor blood cells express Kit (otherwise known as the Stem Cell Factor (SCF) receptor or Kit ligand receptor), mature blood cells express Tim-3, and malignant blood cells express both receptors.

It is difficult to determine the relative expression of such biomarkers accurately. In addition, such diagnostics require the use of significantly costly quantitative techniques and equipment such as flow cytometry to look at cell numbers and morphology. Furthermore, in some leukaemias, there is a paradox in that numbers of a particular cell type may actually be lower than the normal range, so flow cytometry is not always informative.

Thus, there is a continuing need for faster and more accurate tools to enable the correct and speedy diagnosis of leukaemia, especially acute leukaemias.

WO 2014/168992 discloses LPHN1 as being one of 170 GPCRs expressed in indolent CLL cells. WO 2013/169771 discloses the use of a mutation in LPHN1 to diagnose a relapse in leukaemia. Silva et al (PNAS (July 2011) 108(29); 12113-12118) describes investigations into the nature of the interaction between LPHN1 and its endogenous receptor, with a focus on expression in the brain and a role for the ligand and receptor in the fields of schizophrenia, anxiety and attention deficit/hyperactivity disorder.

The inventors have found that the latrophilin family of G-protein-coupled receptors provide a clear indicator of leukaemia cells found in blood. Thus, the present invention resides in the use of latrophilin expression as a biomarker for the diagnosis of leukaemia in a subject.

Latrophilins form a group of structurally similar adhesion G-protein-coupled receptors (GPCRs) that function as exocytosis promoters acting through calcium mobilisation/signalling machinery (Capogna M. et al (2003) J. Neurosci. 23 4044-4053; Volynski K.E. et al(2004) EMBO J. 23 4423-4433; Ushkaryov Y.A. et al (2008) Pharmacology of Neurotransmitter Release 184 171-206; Silva J.-P. et al (2009) J. Neurochem. 111 275-290; Silva J.-P. and Ushkaryov Y. (2010) Adhesion-GPCRs: Structure to Function. 59-75). This group contains three proteins with 48-63% of structural homology, known as latrophilin 1 (LPHN1), latrophilin 2 (LPHN2) and latrophilin 3 (LPHN3). Initially, LPHN1 and LPHN3 were detected only in neurons (Davletov B.A. et al (1996) J. Biol. Chem. 271 23239-23245; Lelianova V.G. et al (1997) J. Biol. Chem. 272 21504-21508). However, LPHN2 is more ubiquitously expressed and its role in normal and pathological processes has been discussed (Sugita S. et al(1998) J. Biol. Chem. 273 32715-32724; Matsushita H. et al (1999) FEBS Lett. 443 348-352; White G. R.M. et al (1998) Oncogene 17 3513-3519; Bushel P.R. et al (2012) PLoS ONE 7 e34286; Zhang S. et al(2014) PLoS ONE 9 e91466). In addition, LPHN3 has also recently been linked to some cancers (Kan Z. et al(2010) Nature 466 869-875; Kotepui M. et al(2012) Asian Pacific J. Cancer Prev. 13 5879-5882), while LPHN1 has been found in non-small cell lung cancer (Hsu Y.-C. et al(2009) Clin. Cancer Res. 15 7309-7315). However, the expression of latrophilin in malignant myeloid leucocytes has not been investigated or even profoundly discussed.

The inventors have found that latrophilin is not expressed in healthy human leucocytes, even at the mRNA level, and expression is not induced by pro-inflammatory agents and growth factors in healthy white blood cells.

Functionally, activation of latrophilin induces intracellular calcium mobilisation, thereby significantly promoting exocytosis. Exocytosis is crucial not only for neuronal function but also for the formation of myeloid cells, a process termed "haematopoiesis" that includes the proliferation and differentiation of stem cells into blood cells.

Exocytosis becomes vital for myeloid leucocyte survival, proliferation and bone marrow angiogenesis upon their malignant transformation. First of all, release of vascular endothelial growth factor (VEGF) and other pro-angiogenic factors is required for bone marrow angiogenesis. Bone marrow vasculature plays a pivotal role in haematopoiesis under leukaemia conditions. Secondly, production and secretion of interleukin 6 (IL-6) is required to promote survival and proliferation of leukaemia cells. These processes require efficient exocytosis but the functional capabilities of leukaemia cells are normally very limited. Therefore, myeloid leukaemia cells tend to activate the expression of certain genes encoding highly active exocytosis receptors, such as latrophilin family members.

Exocytosis plays a pivotal role in myeloid leukaemia progression. The physiological environment of leukaemia cells is normally hypoxic (for example, in bone marrow), caused by a constantly increasing number of cells, while the amount of blood vessels remains unchanged. As part of their long-term adaptation, leukaemia cells require increased angiogenesis, which may be induced by major angiogenic growth factors such as VEGF and erythropoietin (EPO). Malignant leucocytes release angiogenic growth factors through exocytosis. In addition, these cells secrete intercellular signalling mediators that stimulate the production of growth factors promoting leukaemia cell proliferation. Thus, exocytosis determines the ability of liquid tumour (leukaemia) cells to survive and proliferate in the long-term.

It will be appreciated that the hematopoietic cell cancer may be of myeloid or lymphoid origin. In other words, the cancerous cells may be of myeloid or lymphoid lineage.

The present invention is particularly applicable to leukaemia. The leukaemia may be a myeloid leukaemia (including mast cell malignancies) or an acute leukaemia. In the alternative, the leukaemia may be a lymphoid leukaemia or a chronic leukaemia. In another alternative, the haematopoietic cell cancer may be a mast cell leukaemia or a mast cell sarcoma.

While LPHN1 has been directly linked to exocytosis, expression of LPHN2 and LPHN3 may also be suitable for use as a biomarker for the diagnosis of haematopoietic cell cancer and it will be appreciated that expression of any combination of the three latrophilin isoforms may also be used.

For example, LPHN1, 2 and/or 3 expression may be used to detect and diagnose AML, while LPHN1 may be used for the detection and diagnosis CLL and LPHN1 and/3 may be used to detect malignant mast cells.

The present invention also encompasses a method for the diagnosis of haematopoietic cell cancer in a subject, wherein the method comprises detecting one or more latrophilin isoforms on white blood cells.

Expressed in another way, the invention encompasses a method of detecting haematopoietic cell cancer, the method comprising:
a) obtaining a blood sample from a human patient;
b) detecting whether one or more latrophilin isoforms are expressed on white blood cells from the blood sample by contacting the white blood cells with an antibody or ligand and detecting binding between the one or more latrophilin isoforms and the antibody or ligand.

As set out above, it is preferred if one or more of LPHN1, LPHN2 and LPHN3 is detected. Techniques and methods for the detection and visualisation of binding occurrences are well known in the art and any suitable method may be selected. For example, detection may be by way of a suitable antibody for which latrophilin is an antigen, or a latrophilin ligand such as Lasso (also known as teneurin-2), alpha-latrotoxin or LTX^{N4C}. An antibody or ligand ideally includes a detectable marker such as a radio label or fluorophore.

It will be understood that the white blood cells are obtained from a blood sample taken from the subject. Thus, the method is an *ex vivo* method. Ideally the white blood cells are separated from whole blood, for example by density gradient centrifugation of anti-coagulated whole blood and separation and selection of the buffy coat layer.

In one embodiment of the method of the present invention, white blood cells expressing latrophilin may be captured using a latrophilin capture agent, such as an antibody against latrophilin. The antibody may be specific for LPHN1, LPHN2 and/or LPHN3 or may be able to bind to more than one latrophilin isoform.

Once captured, any cells expressing latrophilin will carry a new label in the form of the latrophilin capture agent. These labelled cells may now be visualised by any suitable methods, for example using flow cytometry such as Fluorescence-activated cell sorting (FACS). It will be appreciated that such techniques may include the use of secondary labels or antibodies against latrophilin antibodies or ligands, or the primary antibodies or ligands may carry a label such as a radio or fluorescent label. Alternatively, the captured cells may be visualised using known agents suitable for cell detection, such as an antibody against a cell surface protein or dyes such as lipid partitioning dyes (including Dil and DiO).

Ideally, the latrophilin capture agent is coated onto a surface, such as an internal wall of at least one well of a microtitre plate. Cells that do not express latrophilin on their surface may then be washed off and discarded, leaving latrophilin-expressing cells bound to the coated surface *via* the capture agent.

A microtitre plate, also known as a microplate, microwell plate or multiwell plate, is a flat plate with multiple "wells" that are used as small test tubes. The microplate has become a standard tool in analytical research and clinical diagnostic testing laboratories. A very common usage is in the enzyme-linked immunosorbent assay (ELISA), which is the basis of most modern medical diagnostic testing in humans and animals.

A microplate typically has 6, 24, 96, 384 or even 1536 sample wells arranged in a 2:3 rectangular matrix. Some microplates have even been manufactured with 3456 or 9600 wells and an "array tape" product has been developed that provides a continuous strip of microplates embossed on a flexible plastic tape. Each well of a microplate typically holds between tens of nanolitres to several millilitres of liquid. Wells may be either circular or square, conical or flat-bottomed.

In addition, other methods of selecting haematopoietic cells, such as leukaemia cells, may be used where cells expressing latrophilin isoforms may be attached to material surfaces, including but not limited to surface plasmon resonance chambers, gel beads, paper strips or nanomaterials.

While detecting the presence of latrophilin isoforms on white blood cells is believed to be sufficient to ascertain whether or not a subject has haematopoietic cell cancer, the level of expression may not always be high enough to be detected. Thus, it may be desirable to enhance expression of latrophilin isoforms in the white blood cells before detection.

Accordingly, in one embodiment, the method further comprises enhancing latrophilin expression on the white blood cells. If any healthy cells are captured in error, latrophilin expression will only be enhanced on malignant cells because healthy cells are not capable of expressing latrophilin.

Latrophilin expression on the malignant white blood cells may be enhanced using a pro-inflammatory stimulus. Suitable stimuli include growth factors, cytokines, haematopoietic agents and pro-inflammatory ligands.

A suitable growth factor is ideally a natural hematopoietic factor or a cytokine, which up-regulates cell proliferation. For example, SCF plays an important role in haematopoiesis. SCF binds Kit (CD117) and also acts as a growth factor. Suitable pro-inflammatory cytokines include interleukin-6 (IL-6), interleukin-1beta (IL-1 beta) and tumour necrosis factor alpha (TNF-alpha).

Pro-inflammatory ligands include ligands for toll-like receptors (TLR). Toll-like receptor 4 (TLR4) detects lipopolysaccharide from Gram-negative bacteria and plays a fundamental role in pathogen recognition and activation of innate immunity. A suitable ligand for TLR4 may be lipopolysaccharides (LPS) or its endogenous ligands including hyaluronic acid-binding protein (HABP) or high-mobility group protein B1 (HMGB-1).

LPS's are large molecules consisting of a lipid and a polysaccharide that are found in the outer membrane of Gram-negative bacteria and elicit strong immune responses in animals. While any suitable source of LPS may be used, LPS derived from *Pseudomonas aeruginosa* has been found to be particularly suitable in the present application.

The increased expression of certain cell-surface receptors is cancer cell-specific, so such proteins could be considered as haematopoietic cell and leukaemia antigens. One such antigen is the T cell immunoglobulin and mucin domain 3 (Tim-3). This protein is expressed by T cells, monocytes, dendritic cells, mast cells and microglia. However, during malignant transformation, Tim-3 expression in myeloid cells is highly increased. It has been found that, in myeloid cells (unlike in T cells), ligand-dependent Tim-3 activation triggers TNF-α generation and secretion suggesting similarities in Tim-3-dependent and pro-inflammatory signalling (Anderson A.C. et al (2007) Science 318 1141-1143).

In other words, latrophilin expression on malignant white blood cells may be induced using a pro-inflammatory factor that may also be described as a pro-leukaemic factor and an enhancer of exocytosis. The factor stimulates the biological response of white blood cells which, under leukaemic condition, enhances exocytosis of the cells. This allows the cells to adapt to low oxygen availability caused by increasing the number of malignant cells and thus reducing the amount of oxygen supplied to each cell. Exocytosis of pro-angiogenic factors induces the generation of neovasculature while cytokines activate glucose mobilisation making sugar available to malignant cells which become dependent on this anaerobic pathway under low oxygen availability.

In another embodiment of the method, the presence of latrophilin on white blood cells may be detected by stimulating exocytosis of the white blood cells and detecting one or more proteins, or fragments thereof, released from the white blood cells as a result of exocytosis. In this way, the occurrence of false positive and negative readings is further reduced and confidence in the detection of latrophilin expression is enhanced.

Exocytosis is a durable, energy-consuming process by which a cell directs the contents of secretory vesicles out of the cell membrane and into the extracellular space. These membrane-bound vesicles contain soluble proteins to be secreted to the extracellular environment, as well as membrane proteins and lipids that are sent to become components of the cell membrane.

Ideally, in the method of the present invention, exocytosis is triggered by the influx of calcium and is preferably specifically stimulated by one or more latrophilin ligands. Suitable latrophilin ligands (agonists) may be selected from the group comprising: a latrophilin antibody, alpha-latrotoxin and its mutants, and Lasso/teneurin-2 (Volynski K.E. et al (2004) EMBO J. 23, 4423-4433; Silva J.-P. et al (2009) J Biol Chem 284 6495-6506).

Proteins, or fragments thereof, released from the white blood cells as result of exocytosis include cytokines, hormones and growth factors and may be detected by any suitable means, including detecting the binding of one or more of an antibody, a radio label (isotopic label or isotopic marker) and/or fluorophore.

Disclosed is also a kit for the detection of one or more latrophilin isoforms on white blood cells collected from a subject. The kit comprises:
i) a latrophilin capture agent;
ii) one or more factor to enhance latrophilin expression;
iii) one or more latrophilin ligand; and
iv) means to visualise products of exocytosis from the white blood cells.

Either the white blood cells may be captured while in suspension, or a surface may be coated with the capture agent. A particularly suitable coated surface may be an internal surface of a well in a microtitre plate.

Ideally, the latrophilin capture agent is an antibody against latrophilin. Such an antibody may be specific for LPHN1, LPHN2 or LPHN3 or any combination thereof.

In one example, the kit optionally comprises one or more reagent to visualise the captured cells. The one or more reagent may enable visualisation of the captured cells in suspension, or when the capture agent is coated on a surface.

The one or more factor to enhance latrophilin expression is a growth or inflammatory stimulus as defined hereinabove. Suitable factors include stem cell factor and LPS derived from *Pseudomonas aeruginosa.*

Ideally, the one or more latrophilin ligand (agonist) is selected from the group comprising: a latrophilin antibody, alpha-latrotoxin and Lasso/teneurin-2.

It will be appreciated that the products of exocytosis may be visualised by any suitable means, such as one or more of an antibody or a radio- or fluorophore-labelled ligand.

In a yet further aspect, the present disclosure encompasses uses and methods as set out above to monitor the effectiveness of therapy to treat or slow the progression of haematopoietic cell cancer such as leukaemia, or to decide on initiation, continuation or discontinuation (ending) of the therapy.

Therapy encompasses any suitable treatment, including but not limited to chemotherapy, radiotherapy, bone marrow and/or stem cell transplant and/or one or more agent to stimulate white blood cell and/or stem cell production in the body, steroids and new chemical, biochemical or biological entities.

The present invention will now be described in more detail with reference to the following figures in which:

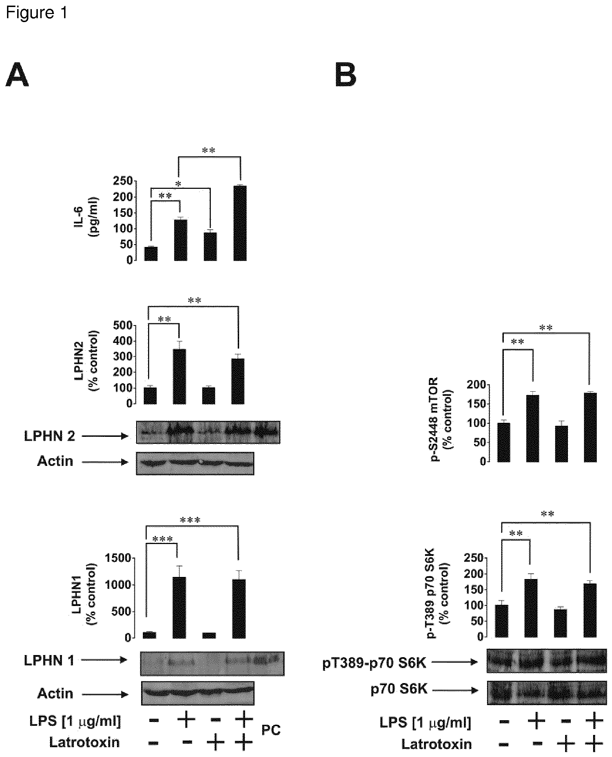

**Figure 1** shows the effects of LPS and alpha-latrotoxin (LTX) on LPHN1/2 expression, IL-6 exocytosis and mTOR activity in U937 human AML cells. U937 cells were exposed to 1 µg/ml LPS, 500 pM LTX or a combination of these two ligands for 24 h. Following cell lysis, latrophilin protein levels were analysed by Western blot. IL-6 release was measured by ELISA **(A).** Phospho-S2448 mTOR and phospho-T389 p70 S6K1 were detected using ELISA and Western blot respectively **(B).** Western blot images show a representative experiment of six, which gave similar results. Data are mean values ± SD (n=6). * - p < 0.05; ** - p < 0.01; *** - p < 0.001.

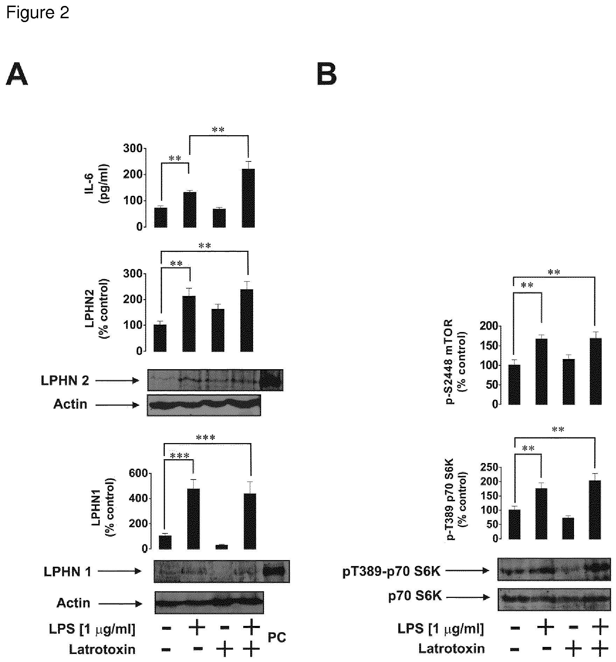

**Figure 2** shows the effects of LPS and LTX on LPHN1/2 expression, IL-6 exocytosis and mTOR activity in THP-1 human AML cells. THP-1 cells were exposed to 1 µg/ml LPS, 500 pM LTX or a combination of these two ligands for 24 h. Following cell lysis, latrophilin protein levels were analysed by Western blot. IL-6 release was measured by ELISA **(A).** Phospho-S2448 mTOR and phospho-T389 p70 S6K1 were detected using ELISA and Western blot respectively **(B).** Western blot images show a representative experiment of six which gave similar results. Data are mean values ± SD (n=6). * - p < 0.05; ** - p < 0.01; *** -p < 0.001.

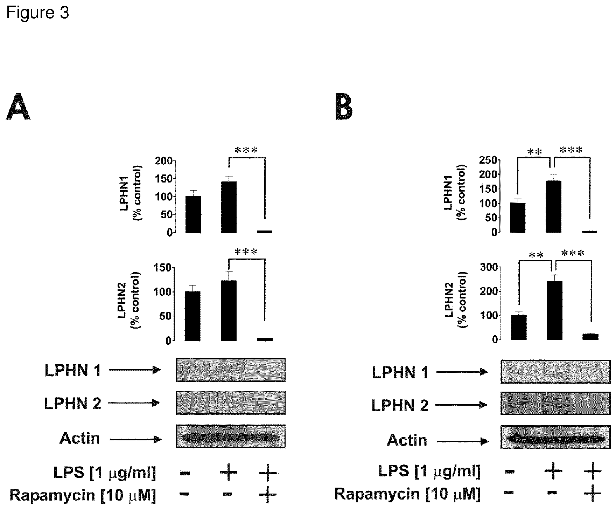

**Figure 3** shows that expression of LPHN1/2 proteins in human AML cell lines is an mTOR-dependent process. U937 **(A)** and THP-1 **(B)** cells were exposed to 1 µg/ml LPS for 4 h with or without 1 h pre-treatment with 10 µM rapamycin. Latrophilin protein levels were analysed by Western blot. Western blot images show a representative experiment of six which gave similar results. Data are mean values ± SD (n=6). * - p < 0.05; ** - p < 0.01.

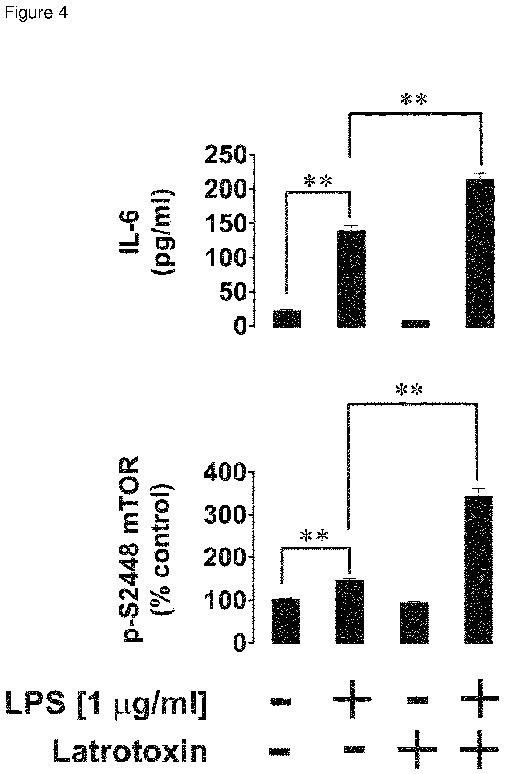

**Figure 4** shows the effects of LPS and LTX on IL-6 exocytosis and mTOR activity in primary human AML cells. Primary human AML-PB001F cells were exposed to 1 µg/ml LPS, 500 pM LTX or a combination of these two ligands for 24 h. Following cell lysis, IL-6 release was measured by ELISA. Phospho-S2448 mTOR levels in cell lysates were also characterised using ELISA. Data are mean values ± SD (n=6). * - p < 0.05; ** - p < 0.01.

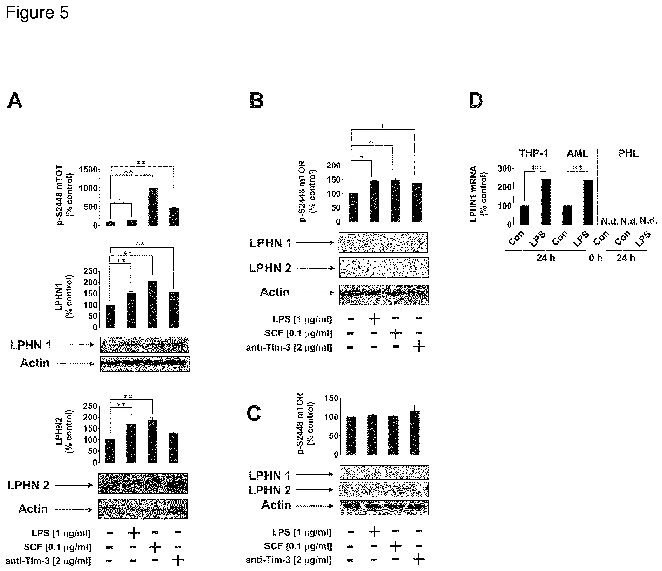

**Figure 5** shows that primary human AML cells, but not healthy primary human leukocytes, express functional LPHN 1 and LPHN 2. Primary AML-PB001F cells were exposed for 4h **(A)** and healthy human leukocytes for 4 h **(B)** or 24 h **(C)** to indicated concentrations of LPS, SCF and anti-Tim-3. Following cell lysis, latrophilin protein levels were analysed by Western blot. Phospho-S2448 mTOR was measured by ELISA. Furthermore, non-treated THP-1 cells, primary human AML cells and primary healthy human leukocytes, as well as those stimulated for 24 h with 1 µg/ml LPS were subjected to quantitative real time polymerase chain reaction qRT-PCR **(D).** Images show one representative result of at least four experiments, which gave similar results. Data are mean values ± SD (n=4). * - p < 0.05; ** - p < 0.01.

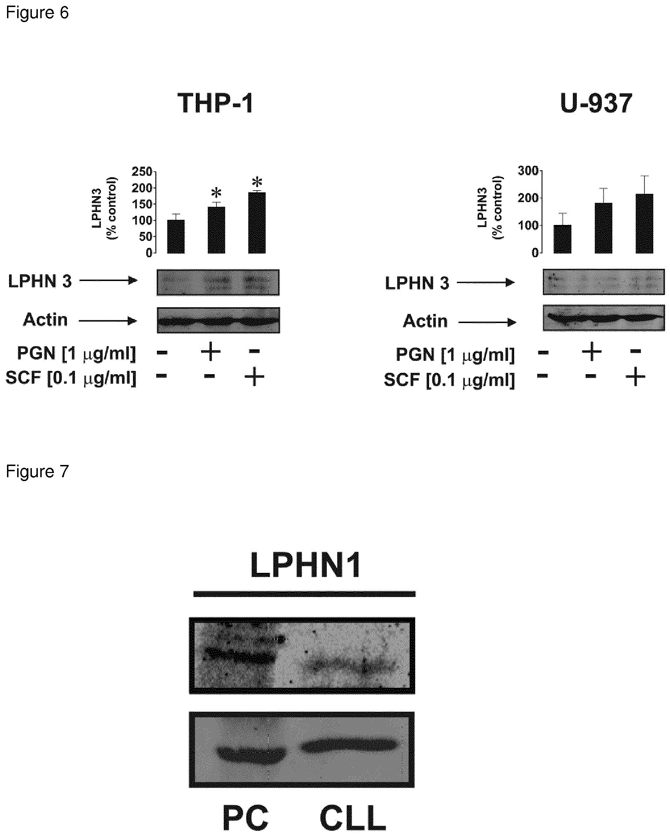

**Figure 6** shows the expression of LPHN3 in THP-1 and U-937 ML cell lines in the absence and presence of either (1) peptidoglycan (PGN), which is a ligand of Toll-like receptor 2 (TLR2), or (2) stem cell factor (SCF). Cells were exposed to the indicated concentrations of stimuli for 4 h, followed by Western blot analysis of LPHN3 expression. Images are from one experiment representative of five independent experiments all of which gave similar results. Quantitative data are the mean values ± SEM (n=5).
**Figure 7** is a Western Blot showing the expression of LPHN1 in primary human chronic lymphocytic leukaemia cells (PC, positive control; rat brain extract). Images are from one experiment representative of three independent experiments which gave similar results.

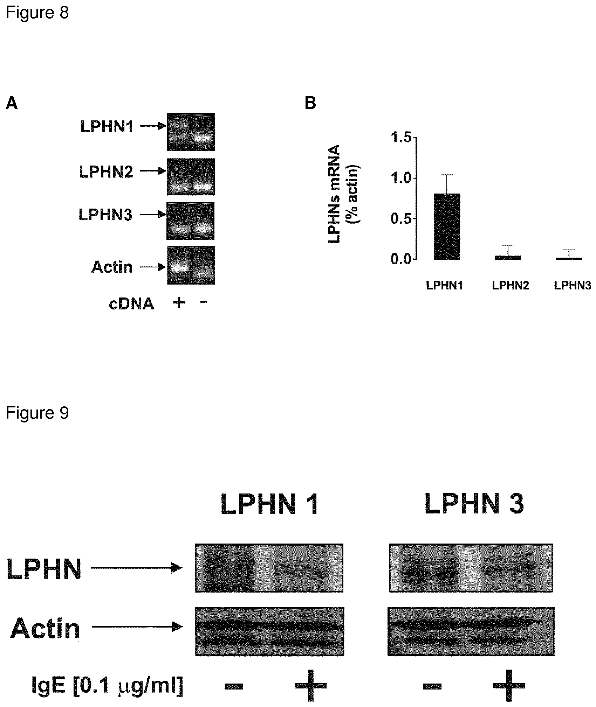

**Figure 8** illustrates the mRNA levels of LPHNs 1, 2 and 3 expressed in primary human chronic lymphocytic leukaemia mononuclear cells (CLL-BM001M, provided by AllCells) isolated from the bone marrow of a patient with chronic lymphocytic leukaemia. **A,** Agarose gel electrophoresis of amplified gene fragments, after 50 PCR cycles. Arrows indicate the expected positions of specifically amplified fragments. Bottom bands, unused oligonucleotide primers. Amplification in the absence of cDNA was used as a negative control. The β-actin gene was used as a house-keeping gene for quantitative analysis of relative gene expression. **B,** Quantitative analysis of the expression of respective genes. The data are shown as mean values ± SEM from three independent qRT-PCR experiments (n = 3), normalised to the expression of β-actin. Only mRNA of LPHN1 was clearly identified, while LPHN2 and LPHN3 mRNAs were undetectable.
**Figure 9** is a Western Blot showing the expression of LPHNs 1 and 3 in a human cell line derived from malignant mast cells. Images are from one experiment representative of three independent experiments which gave similar results.

### Example 1

The following example demonstrates that latrophilin (LPHN) isoforms 1 and 2 are expressed in human acute myeloid leukaemia (AML) cell lines (U937 and THP-1) in an mTOR-dependent manner and are capable of enhancing IL-6 exocytosis.

### Materials and Methods

RPMI-1640 medium, foetal calf serum and supplements were purchased from Sigma (Suffolk, UK). Maxisorp™ microtitre plates were obtained from Nunc (Roskilde, Denmark) as well as from Oxley Hughes Ltd (London, UK). Mouse monoclonal antibodies to mTOR and β-actin, as well as rabbit polyclonal antibodies against phospho-S2448 mTOR, were obtained from Abcam (Cambridge, UK). Antibodies against phospho-T389 and total p70 S6 kinase 1 (p70 S6K1) were obtained from Cell Signalling Technology (Danvers, MA USA). Goat anti-mouse and goat anti-rabbit fluorescence dye-labelled antibodies were obtained from Li-Cor (Lincoln, Nebraska USA). ELISA-based assay kits for the detection of IL-6 were purchased from R&D Systems (Abingdon, UK). LTX was isolated from the venom of black widow spider *Latrodectus sp.* as described by Ashton A.C. et al (Biochimie (2000) 82 453-468). All other chemicals were of the highest grade of purity and commercially available.

THP-1 and U937 human leukaemia monocytic macrophages were obtained from the European Collection of Cell Cultures (Salisbury, UK). Cells were cultured in RPMI 1640 media supplemented with 10% foetal calf serum, penicillin (50 IU/ml) and streptomycin sulphate (50 µg/ml).

Levels of LPHN 1 and LPHN 2, total and phospho-T389 p70 S6K1 were determined by Western blot analysis and compared to β-actin in order to determine equal protein loading, as described by Yasinska I.M. et al (Cell. Mol. Life Sci. (2014) 71 699-710). Li-Cor goat secondary antibodies, conjugated with fluorescent dyes, were used according to the manufacturer's protocol to visualise the proteins of interest using a Li-Cor Odyssey imaging system. Western blot data were analysed quantitatively using Odyssey software and values were normalised against respective β-actin bands.

Phosphorylation of mTOR was monitored using ELISA assays as recently described (Yasinska I.M. *et al* (*supra*); Abooali M. et al (2014) Sci. Rep. 4 6307; Prokhorov A. *et al* (*supra*)). Briefly, ELISA plates were coated with mouse anti-mTOR antibodies and blocked with 2 % BSA. Cell lysates were then added to the wells and kept at room temperature for at least 2 h (under constant agitation). After extensive washing with TBST buffer (Tris-Buffered Saline and Tween 20), anti-phospho-S2448 mTOR antibody was added and plates were incubated for at least 2 h at room temperature with constant agitation. Plates were then washed with TBST buffer and incubated with 1:1000 Horse Radish Peroxidase-labelled goat anti-rabbit IgG in TBST buffer. After extensive washing with TBST, bound secondary antibodies were then detected by the peroxidase reaction (ortho-phenylenediamine/H₂O₂).

Concentrations of IL-6 released into the cell culture media were analysed by ELISA (R&D Systems assay kit) according to the manufacturer's protocol.

Each experiment was performed at least three times and statistical analysis was conducted using a two-tailed Student's t test or, where appropriate, a one-way ANOVA with post-hoc Tukey test, with correction for multiple pairwise comparisons.

### Results

Expression of LPHN 1 and LPHN 2 in human AML cell lines - U937 and THP-1 - was investigated. Cells were stimulated for 24 h with 1 µg/ml LPS, 500 pM LTX or a combination of these ligands. LPS is a pathogen-associated molecular pattern shared by Gram-negative bacteria and is recognised by the Toll-like receptor 4 (TLR4), which is highly expressed by human myeloid cells. Importantly, LPS was chosen because TLR4 may be targeted by endogenous ligands (like proteins released by dysfunctional mitochondria) to promote expression/release of IL-6 and other important cytokines/growth factors required for leukaemia cell survival.

It was found that both U937 and THP-1 cells expressed detectable levels of LPHN 1 and LPHN 2 (Figure 1A and Figure 2A). Commercially available mouse brain extract was used as a positive control. In both U937 and THP-1 cells, LPHN1/2 expression levels were significantly up-regulated by LPS but not by LTX (Figure 1A and Figure 2A). However, the IL-6 exocytosis induced by LPS was highly up-regulated by LTX in both U937 and THP-1 human AML cells (Figure 1A and Figure 2A).

It was also found that LPS, but not LTX, significantly activated the mTOR pathway, increasing its activating phosphorylation at position S2448 and also the phosphorylation of its substrate, p70 S6 kinase 1 (p70 S6K1) at position T389. This was clearly observed in both cell lines (Figure 1B and Figure 2B).

Since mTOR is a master regulator of myeloid cell translation, the role of the mTOR pathways in LPS-induced up-regulation of LPHN1/2 protein levels was investigated.

Both U937 and THP-1 cells were exposed to 1 µg/ml LPS for 4 h with or without 1 h pre-treatment with 10 µM rapamycin (a highly specific mTOR inhibitor). The results show that, unlike 24 h stimulation, 4 h exposure to LPS led to a moderate increase in LPHN1/2 expression in both U937 and THP-1 cells. However, the effect was stronger in THP-1 cells. Rapamycin attenuated the expression of LPHN 1 and LPHN 2 in both cell lines (Figure 3 A and B). This suggests that latrophilin protein accumulation strongly depends on the mTOR pathway which displays a clear background activity in resting cells and is further increased upon stimulation (for example LPS, Figures 1B and 2 B).

### Conclusion

The results demonstrate that resting AML cells from the U937 and THP-1 human cell lines express both LPHN 1 and LPHN 2. The expression was up-regulated by LPS *via* the mTOR pathway (a master regulator of myeloid cell translational pathways). LTX, a specific high affinity latrophilin ligand which causes an increase in cytosolic calcium and massive exocytosis in neurons, significantly enhanced LPS-induced exocytosis of IL-6 in both cell lines. Since the production of IL-6 protein depends on mTOR activation, and activity of the mTOR pathway was not up-regulated by LTX in these cell lines, it is clear that LTX was up-regulating IL-6 exocytosis and not its biosynthesis.

Interestingly, both LPHN 1 and LPHN 2 protein levels were nearly abolished when the cells were pre-treated with the mTOR inhibitor rapamycin before exposure of both U937 and THP-1 cells to LPS. This observation suggests that the process of LPHN1/2 expression strongly depends on the mTOR pathway. As demonstrated in the Figures 1B and 2B, in both cell lines there is a background activity of the mTOR pathway which is further up-regulated by LPS.

### Example 2

The following example demonstrates that functional LPHN 1 and LPHN 2 are expressed in primary human AML cells but not in primary healthy human leukocytes.

### Materials and Methods

Unless indicated otherwise, materials and methods were identical to those used in Example 1.

Primary human AML mononuclear cells (AML-PB001 F, newly diagnosed/untreated) were purchased from AllCells (Alameda, CA, USA) and handled in accordance with manufacturer's instructions.

Primary human leukocytes were obtained from buffy coat blood (prepared from healthy donors) purchased from the National Health Blood and Transfusion Service (NHSBT, UK) following ethical approval (REC reference: 12/WM/0319). Mononuclear-rich leukocytes were obtained by Ficoll-density centrifugation according to the manufacturer's protocol. Cell numbers were determined using a haemocytometer and diluted accordingly with HEPES-buffered Tyrode's solution before treatment as indicated.

Human Stem Cell Factor (SCF) protein was produced in *E. coli* and purified in accordance with published protocols (Wang C. et al (2008) Appl. Biochem. Biotechnol. 144 181-189).

To obtain anti-TIM3 mouse monoclonal antibodies, human TIM-3 Ig-like V-type domain (extracellular domain, position 22-124) was produced in *E. coli*, refolded from inclusion bodies and purified according to standard protocols. Monomer and aggregates of the protein were collected and used to immunise 8 week old C56BL/6 mice (5 for protein monomer and 5 for aggregates) using standard protocols for mouse immunisation. Spleens from highly responsive mice were collected and used to create hybridomas. Antibodies were screened according to their affinity to the Tim-3 monomer (*via* ELISA and SPR). The highest affinity monoclonal antibody was selected and used in this study (Prokhorov A. *et al* (*supra*))*.*

### Results

Experiments were carried out to ascertain whether or not LPHN 1 and LPHN 2 are expressed and function in primary human AML cells. AML-PB001 F primary human mononuclear blasts were used and exposed for 24 h to 1 µg/ml LPS, 500 pM LTX or a combination of these ligands.

It was found that LPS up-regulated both mTOR activation and IL-6 release by these cells. LTX alone did not influence these processes but, in combination with LPS, significantly up-regulated both mTOR activation and IL-6 exocytosis (Figure 4).

To analyse LPHN1/2 expression levels in AML-PB001 F cells, the cells were exposed to mTOR activators (1 µg/ml LPS, 0.1 µg/ml SCF or 2 µg/ml anti-Tim-3 antibody) for 4 h. It was found that each stimulus significantly increased phosphorylation of S2448 in mTOR and therefore mTOR activation (Figure 5A). SCF induced the strongest effect due to a high level of Kit (SCF receptor) expressed by these cells. All stimuli also significantly up-regulated LPHN1/2 expression as detected by Western blot analysis.

LPS and SCF significantly up-regulated LPHN2 expression in AML-PB001 F primary cells, while LPHN2 expression was non-significantly up-regulated by anti-Tim-3 (Figure 5A). This observation is in line with results reported by others on the intensity of mTOR-dependent effects provoked by LPS, SCF and anti-Tim-3 in human AML cells (Prokhorov, A. *et al*(*supra*)).

Quantitative real-time PCR (RT-PCR) experiments suggested that THP-1 cells and primary AML cells, but not healthy primary human leukocytes, produce LPHN1 mRNA (Figure 5D). Furthermore, 1 µg/ml LPS was able to upregulate LPHN1 mRNA levels slightly in THP-1 cells. However, no significant changes were observed after 24 h exposure. Intriguingly, 24 h stimulation of primary AML cells with 1 µg/ml LPS led to a significant increase in LPHN1 mRNA levels. In primary healthy human leukocytes even 24 h exposure to 1 µg/ml did not induce LPHN1 mRNA expression (Figure 5D).

### Conclusion

These experiments demonstrate that similar effects may be observed in primary human AML cells, using primary AML-PB001 F mononuclear blasts obtained from leukaemia patients, to the effects seen in AML cell lines. It has been found that resting primary human AML cells and those exposed to mTOR stimuli (LPS, SCF and anti-Tim-3) express both LPHN1 and LPHN2. mTOR activators were able to upregulate the levels of both proteins in primary AML cells, as also seen in cell lines. LTX increased LPS-induced IL-6 release.

However, LTX also up-regulated LPS-induced mTOR-activating phosphorylation in AML cells, while it failed to do so in the cell lines. This is likely to be a result of low expression of TLR4 (LPS receptor) in AML cells, as demonstrated by the provider of the AML cells. In these cells, LPS binding to TLR4 causes some mTOR activation via phosphorylation of its S2448, but a large proportion of mTOR molecules remains unphosphorylated/inactive and any additional activation of the mTOR signalling mechanisms may still be able to increase mTOR activation. In cell lines, where TLR4 expression is much higher, LPS-induced mTOR activation may be approaching its maximum and LTX may not be able to increase mTOR activation further.

Importantly, primary leucocytes obtained from healthy donors (buffy coats) did not express any latrophilin. Furthermore, this expression was not inducible by 4 or 24 h exposure of the cells to any of the stimuli which showed positive effects in AML cells (LPS, SCF or anti-Tim-3). This means that expression of functional LPHN1 and LPHN2 is specific only to leukaemia cells.

An important observation was also made in quantitative RT-PCR experiments, which have demonstrated that healthy primary human leukocytes do not produce LPHN1 mRNA. This means that a basic protein expression profile change is taking place in leukocytes during malignant transformation. The LPHN1 gene appears to be repressed in leukocytes. However, LPHN1 expression does take place in malignantly transformed cells. The value of this protein to malignant leucocytes is obvious - these are weak cells that require production of certain growth factors (like VEGF) and cytokines (including IL-6) to survive and progress the disease. However, leucocyte exocytosis is an energy-consuming process associated with major cytoskeleton alterations. This could be beyond the capabilities of malignant cells. Therefore, they are likely to require powerful mechanisms inducing exocytosis, like those available in neurons.

### Example 3

The following example demonstrates LPHN3 protein expression in human acute myeloid leukaemia cells.

### Materials and Methods

THP-1 human myeloid leukaemia monocytes and U937 human myeloid monocytes were obtained from the European Collection of Cell Cultures (Salisbury, UK). Cells were cultured in RPMI 1640 media supplemented with 10% foetal bovine serum, penicillin (50 IU/ml) and streptomycin sulphate (50 µg/ml).

THP-1 and U-937 cells were exposed for 4 h to indicated concentrations of peptidoglycan (PGN, Sigma Aldrich, UK), or stem cells factor (SCF, Human SCF was a kind gift of Dr. Luca Varani, IRB, Bellinzona, Switzerland). Non-treated cells incubated under similar conditions were used as a control. Cells were then harvested, lysed (whole cell extracts were prepared using 50 mM Tris-HCI, 5 mM EDTA, 150 mM NaCl, 0.5% Nonidet-40, 1 mM PMSF buffer) and subjected to Western blot analysis of LPHN3.

The levels of the LPHN3 isoform was analysed using Western blot. The polyclonal rabbit anti-peptide antibodies PAL1, PAL2 and PAL3 against LPHN1, 2 and 3 respectively have been produced in-house and previously described (Davydov et al (2009) Bull. Exp. Biol. Med. 148: 869-873; Silva et al (2011) Proc. Natl. Acad. Sci. U.S.A. 108: 12113-12118). LPHN3 was determined as described in (Gonçalves Silva et al (2015) Oncotarget 6: 33823-33833; Prokhorov et al (2015) Int. J. Biochem. Cell Biol. 59: 11-20). Fluorescently labelled antibodies (Li-Cor) were used according to the manufacturer's protocol to visualise the proteins of interest using an Odyssey imaging system (Li-Cor). Western blot data were subjected to quantitative analysis using the Odyssey software and values were normalised against respective β-actin bands.

### Results

As can be seen in Figure 6, LPHN3 was expressed in both THP-1 and U-937 human myeloid leukaemia cell lines in a statistically significant amount. THP-1 and U-937 cells express different relative amounts of LPHN3 and LPHN1.

### Conclusion

These results show that LPHN3 is expressed in human myeloid leukaemia cell lines and support a role for this receptor isotype in leukaemia.

### Example 4

In this example, the expression of LPHN isoforms in primary chronic lymphoid leukaemia cells was investigated.

### Materials and Methods

Primary human bone marrow-derived CLL mononuclear cells (CLL-BM001F, newly diagnosed/untreated leukaemia) were obtained from AllCells (Alameda, CA, USA) and handled in accordance with manufacturer's protocol. Cells were analysed following ethical approval (REC reference: 16-SS-033).

The primary malignant mononuclear cells were extracted using a lysis buffer (50 mM Tris-HCI, 5 mM EDTA, 150 mM NaCl, 0.5% Nonidet-40, 1 mM PMSF buffer) and subjected to Western blot analysis of LPHN1, 2 and 3.

The levels of LPHN1, 2 and 3 isoforms were analysed using Western blot. The polyclonal rabbit anti-peptide antibodies PAL1, PAL2 and PAL3 against LPHN1, 2 and 3 respectively have been produced in-house and previously described (Davydov *et al* (*supra*); Silva *et al* (*supra*). Expression levels of LPHNs 1-3 were determined as described in Gonçalves Silva *et al* (*supra*); Prokhorov *et al* (*supra*). Fluorescently labelled antibodies (Li-Cor) were used according to the manufacturer's protocol to visualise the proteins of interest using an Odyssey imaging system (Li-Cor). Western blot data were subjected to quantitative analysis using the Odyssey software and values were normalised against respective β-actin bands.

A separate fraction of cells was used for quantitative real-time PCR (qRT-PCR) to analyse mRNA levels of LPHN isoforms. Total RNA was extracted using the Illustra RNAspin Midi RNA isolation kit (GE Healthcare) and quantified spectroscopically using Nanodrop 2000® (Thermo Scientific). cDNA was then synthesised with the help of Transcriptor First Strand cDNA Synthesis Kit (Roche), which was used in accordance with the manufacturer's protocol. Relative quantification of LPHN mRNAs was performed using SYBR Green I Master reaction mix (Roche) and a LightCycler 480 (Roche). The house-keeping gene β-actin was used as a reference gene in all cases.

The following primers were used at a final concentration of 0.5 µM):

| | |
|---|---|
| LPHN1: | 5'-AGCCGCCCCGAGGCCGGAACCTA-3' (SEQ ID NO:1); |
| | 5'-AGGTTGGCCCCGCTGGCATAGAGGGAGTC-3' (SEQ ID NO:2); |
| LPHN2: | 5'- CACAACGTCGACCTCACACTACCAGTCAAGCCTG-3' (SEQ ID NO:3); |
| | 5'- TGGCACTATTAGAGACTAGTCACCAGCTGCATTTG-3' (SEQ ID NO:4); |
| LPHN3: | 5'- GACCTGGGCCTTTGGACTCATGTA-3' (SEQ ID NO:5); |
| | 5'- CGCCGCTGGCAATGCTGTA-3' (SEQ ID NO:6); |
| Actin: | 5'-TTCGCGGGCGACGATGC-3' (SEQ ID NO:7); |
| | 5'-GGGGCCACACGCAGCTCATT-3' (SEQ ID NO:8). |

PCR reactions were begun with incubation at 95°C for 3 min 30 s, then proceeded for 45 cycles of 95°C for 10 s, 60°C for 20s and 72°C for 10 s. Fluorescence levels were detected at 80°C in each cycle. A final elongation step took place at 72°C for 5 min. Raw fluorescence data were analysed using LinRegPCR quantitative PCR data analysis programme (Ruijter et al (2009) Nucleic Acids Res. 37: e45). Amplified products were validated using 1.5% agarose gel containing ethidium bromide.

### Results

As shown in the Western Blot in Figure 7, only LPHN1 protein was detectable in CLL cells. Similar results were obtained using qRT-PCR in which only the mRNA of LPHN1 was detectable (data not shown). mRNA for LPHNs 2 and 3 was not seen in CLL cells.

Figure 8 shows the mRNA levels of LPHNs1, 2 and 3 in primary human chronic lymphocytic leukaemia (CLL) cells in which only LPHN1, but not LPHN2 or LPHN 3 is expressed. This is seen on both mRNA and protein levels.

### Conclusion

The expression of LPHN1 in CLL cells suggests a possible role for this latrophilin isoform in lymphatic leukaemias.

### Example 5

In this example, the expression of LPHN isoforms in LAD2 human mast cells derived from malignant mast cells originally isolated from a mast cell sarcoma was investigated. Mast cells and myeloid hematopoietic cells are derived from the same type of stem cells (human myeloid progenitor cells). Therefore, both mast cells and myeloid leukocytes are considered to be "myeloid haematopoietic cells". Thus both myeloid leukaemia and mast cell malignancies are present in myeloid cell cancers.

### Materials and Methods

LAD2 mast cells were kindly provided by A. Kirshenbaum and D. Metcalfe (NIH, USA) (Kirshenbaum et al (2003) Leuk. Res. 27: 677-682). Cells were cultured in Stem-Pro-34 serum-free media supplemented with 2 mM L-glutamine, penicillin (100 units/ml), streptomycin (100 µg/ml) and recombinant human SCF (100 ng/ml).

Non-treated LAD2 human malignant mast cells and those exposed for 24 h to 0.1 µg/ml human IgE were lysed (whole cell extract was prepared using 50 mM Tris-HCI, 5 mM EDTA, 150 mM NaCl, 0.5% Nonidet-40, 1 mM PMSF buffer) and subjected to Western blot analysis as described in Example 4.

### Results

As shown in Figure 9, LPHNs 1 and 3 were detectable in malignant mast cells, while LPHN2 was not detectable. Interestingly, the levels of LPHN1 and LPHN3 were decreased in IgE-treated cells. IgE binds to high-affinity IgE receptors (FcεRI) on mast cells and plays a role in their activation during type-I hypersensitivity reactions (allergies) and in certain autoimmune diseases. Actin staining was employed to confirm equal protein loading in each well of the gel.

### Conclusion

LAD2 human malignant mast cells express LPHN1 and LPHN3 proteins, while LPHN2 was not detectable by Western blot analysis.

The results presented here clearly demonstrate that functional LPHN1 and LPHN2 are expressed in human AML cells but not in healthy leucocytes. Furthermore, in healthy leucocytes, LPHN1/2 expression is not inducible. In AML cells, latrophilin is likely to contribute to exocytosis of growth and angiogenic factors required for proliferation of AML cells and bone marrow angiogenesis. LPHN3 is expressed in human myeloid leukaemia cell lines while LPHN1, but not the two other known isoforms, is detectable in human CLL cells. Human malignant mast cells (another type of myeloid haematopoietic cell cancer) express LPHN1 and LPHN3, but not LPHN2.

Therefore, expression of latrophilin isoforms should be suitable as a novel biomarker for haematopoietic cell cancer diagnostics such as leukaemia and provide novel targets for therapy and drug delivery.

## Claims

1. In vitro use of expression of one or more latrophilin isoforms in a sample as a biomarker for the diagnosis of haematopoietic cell cancer in a subject.

2. Use according to Claim 1, wherein the hematopoietic cell cancer is of myeloid or lymphoid origin.

3. Use according to Claim 1 or Claim 2, wherein the hematopoietic cell cancer is leukaemia.

4. Use according to any one of Claims 1 to 3, wherein the leukaemia is a myeloid leukaemia or an acute leukaemia.

5. Use according to any one of Claims 1 to 3, wherein the leukaemia is a lymphoid leukaemia or a chronic leukaemia.

6. Use according to any one of Claims 1 to 4, wherein the hematopoietic cell cancer is a mast cell leukaemia or a mast cell sarcoma.

7. Use according to any one of Claims 1 to 6, wherein latrophilin is selected from the group consisting of: latrophilin 1, latrophilin 2 and latrophilin 3.

8. An in vitro method for the diagnosis of haematopoietic cell cancer in a subject, wherein the method comprises detecting increased expression of one or more latrophilin isoforms on white blood cells in a sample as compared to healthy controls.

9. A method according to Claim 8, wherein one or more of latrophilin 1, latrophilin 2 and latrophilin 3 is detected.

10. A method according to Claim 8 or Claim 9, wherein the method further comprises capturing the white blood cells using a latrophilin capture agent.

11. A method according to Claim 10, wherein the latrophilin capture agent is a latrophilin ligand or an antibody against latrophilin.

12. A method according to Claim 11, wherein the antibody against latrophilin is specific for latrophilin 1, latrophilin 2 and/or latrophilin 3.

13. A method according to any one of Claims 10 to 12, wherein the captured white blood cells expressing latrophilin are visualised.

14. A method according to Claim 13, wherein the captured white blood cells are visualised using any one or combination of a latrophilin ligand or an antibody against latrophilin, an antibody against a cell-surface protein, a radio label, a fluorescent label.

15. A method according to any one of Claims 10 to 14, wherein the latrophilin capture agent is coated onto a surface.

16. A method according to Claim 15, where in the coated surface is an internal wall of at least one well of a microtitre plate.

17. A method according to any one of Claims 8 to 16, wherein the method further comprises enhancing latrophilin expression on the white blood cells.

18. A method according to Claim 18, wherein latrophilin expression is enhanced using one or more of a growth factor, a cytokine, a haematopoietic agent and a pro-inflammatory ligand.

19. A method according to Claim 18, wherein the cytokine is a pro-inflammatory cytokine or a haematopoietic agent.

20. A method according to Claim 18 or Claim 19, wherein the cytokine or haematopoietic agent is Stem Cell Factor (SCF).

21. A method according to Claim 18, wherein the pro-inflammatory ligand is a toll-like receptor 4 (TLR4) specific ligand.

22. A method according to Claim 21, wherein the toll-like receptor 4 specific ligand is lipopolysaccharide (LPS).

23. A method according to Claim 22, wherein the lipopolysaccharide is derived from *Pseudomonas aeruginosa.*

24. A method according to any one of Claims 8 to 23, wherein the method further comprises stimulating exocytosis of the white blood cells and detecting one or more proteins, or fragments thereof, released from the white blood cells as a result of exocytosis.

25. A method according to Claim 24, wherein exocytosis is stimulated by one or more latrophilin ligand.

26. A method according to Claim 25, wherein the one or more latrophilin ligand is selected from the group comprising: a latrophilin antibody, alpha-latrotoxin and Lasso/teneurin-2.

27. A method according to any one of Claims 24 to 26, wherein the one or more proteins, or fragments thereof, released from the white blood cells as result of exocytosis are detected by binding one or more of an antibody, a radio label and/or fluorescent ligand.

28. A method according to any one of Claims 24 to 27, wherein the one or more proteins, or fragments thereof, released from the white blood cells as result of exocytosis include cytokines, hormones and growth factors.

## Patentansprüche

1. *In-vitro*-Verwendung einer Expression einer oder mehrerer Latrophilin-Isoformen in einer Probe als Biomarker für die Diagnose von hämatopoetischem Zellkrebs bei einem Subjekt.

2. Verwendung nach Anspruch 1, wobei der hämatopoetische Zellkrebs myeloischen oder lymphoiden Ursprungs ist.

3. Verwendung nach Anspruch 1 oder 2, wobei der hämatopoetische Zellkrebs Leukämie ist.

4. Verwendung nach einem der Ansprüche 1 bis 3, wobei die Leukämie eine myeloische Leukämie oder eine akute Leukämie ist.

5. Verwendung nach einem der Ansprüche 1 bis 3, wobei die Leukämie eine lymphoide Leukämie oder eine chronische Leukämie ist.

6. Verwendung nach einem der Ansprüche 1 bis 4, wobei der hämatopoetische Zellkrebs eine Mastzellleukämie oder ein Mastzellsarkom ist.

7. Verwendung nach einem der Ansprüche 1 bis 6, wobei Latrophilin aus der Gruppe ausgewählt ist, bestehend aus: Latrophilin 1, Latrophilin 2 und Latrophilin 3.

8. *In-vitro*-Verfahren zur Diagnose von hämatopoetischem Zellkrebs bei einem Subjekt, wobei das Verfahren das Nachweisen einer erhöhten Expression einer oder mehrerer Latrophilin-Isoformen auf weißen Blutkörperchen in einer Probe im Vergleich zu gesunden Kontrollen umfasst.

9. Verfahren nach Anspruch 8, wobei eines oder mehrere von Latrophilin 1, Latrophilin 2 und Latrophilin 3 nachgewiesen werden.

10. Verfahren nach Anspruch 8 oder 9, wobei das Verfahren ferner das Einfangen der weißen Blutkörperchen unter Verwendung eines Latrophilin-Einfangmittels umfasst.

11. Verfahren nach Anspruch 10, wobei das Latrophilin-Einfangmittel ein Latrophilin-Ligand oder ein Antikörper gegen Latrophilin ist.

12. Verfahren nach Anspruch 11, wobei der Antikörper gegen Latrophilin für Latrophilin 1, Latrophilin 2 und/oder Latrophilin 3 spezifisch ist.

13. Verfahren nach einem der Ansprüche 10 bis 12, wobei die eingefangenen weißen Blutkörperchen, die Latrophilin exprimieren, sichtbar gemacht werden.

14. Verfahren nach Anspruch 13, wobei die eingefangenen weißen Blutkörperchen unter Verwendung eines oder einer Kombination eines Latrophilin-Liganden oder eines Antikörpers gegen Latrophilin, eines Antikörpers gegen ein Zelloberflächenprotein, einer Radiomarkierung oder einer Fluoreszenzmarkierung sichtbar gemacht werden.

15. Verfahren nach einem der Ansprüche 10 bis 14, wobei das Latrophilin-Einfangmittel auf eine Oberfläche aufgetragen wird.

16. Verfahren nach Anspruch 15, wobei sich in der beschichteten Oberfläche eine Innenwand von mindestens einer Vertiefung einer Mikrotiterplatte befindet.

17. Verfahren nach einem der Ansprüche 8 bis 16, wobei das Verfahren ferner das Verstärken der Latrophilin-Expression auf den weißen Blutkörperchen umfasst.

18. Verfahren nach Anspruch 18, wobei die Latrophilin-Expression unter Verwendung eines oder mehrerer eines Wachstumsfaktors, eines Zytokins, eines hämatopoetischen Mittels und eines proinflammatorischen Liganden verstärkt wird.

19. Verfahren nach Anspruch 18, wobei das Zytokin ein proinflammatorisches Zytokin oder ein hämatopoetisches Mittel ist.

20. Verfahren nach Anspruch 18 oder 19, wobei das Cytokin oder das hämatopoetische Mittel der Stammzellfaktor (SCF) ist.

21. Verfahren nach Anspruch 18, wobei der proinflammatorische Ligand ein für tollähnlichen Rezeptor 4 (TLR4) spezifischer Ligand ist.

22. Verfahren nach Anspruch 21, wobei der für den tollähnlichen Rezeptor 4 spezifische Ligand Lipopolysaccharid (LPS) ist.

23. Verfahren nach Anspruch 22, wobei das Lipopolysaccharid von *Pseudomonas aeruginosa* stammt.

24. Verfahren nach einem der Ansprüche 8 bis 23, wobei das Verfahren ferner das Stimulieren einer Exozytose der weißen Blutkörperchen und das Nachweisen eines oder mehrerer Proteine oder Fragmente davon umfasst, die infolge der Exozytose aus den weißen Blutkörperchen freigesetzt werden.

25. Verfahren nach Anspruch 24, wobei die Exozytose durch einen oder mehrere Latrophilin-Liganden stimuliert wird.

26. Verfahren nach Anspruch 25, wobei der eine oder die mehreren Latrophilin-Liganden aus der Gruppe ausgewählt sind, umfassend: einen Latrophilin-Antikörper, alpha-Latrotoxin und Lasso/Teneurin-2.

27. Verfahren nach einem der Ansprüche 24 bis 26, wobei das eine oder die mehreren Proteine oder Fragmente davon, die infolge der Exozytose aus den weißen Blutkörperchen freigesetzt werden, durch Binden eines oder mehrerer eines Antikörpers, einer Radiomarkierung und/oder eines fluoreszierenden Liganden nachgewiesen werden.

28. Verfahren nach einem der Ansprüche 24 bis 27, wobei das eine oder die mehreren Proteine oder Fragmente davon, die infolge der Exozytose aus den weißen Blutkörperchen freigesetzt werden, Zytokine, Hormone und Wachstumsfaktoren beinhalten.

## Revendications

1. Utilisation *in vitro* de l'expression d'une ou de plusieurs isoformes de la latrophiline dans un échantillon comme biomarqueur pour le diagnostic du cancer des cellules hématopoïétiques chez un sujet.

2. Utilisation selon la revendication 1, le cancer des cellules hématopoïétiques étant d'origine myéloïde ou lymphoïde.

3. Utilisation selon la revendication 1 ou 2, le cancer des cellules hématopoïétiques étant la leucémie.

4. Utilisation selon l'une quelconque des revendications 1 à 3, la leucémie étant une leucémie myéloïde ou une leucémie aiguë.

5. Utilisation selon l'une quelconque des revendications 1 à 3, la leucémie étant une leucémie lymphoïde ou une leucémie chronique.

6. Utilisation selon l'une quelconque des revendications 1 à 4, le cancer des cellules hématopoïétiques étant une leucémie à mastocytes ou un sarcome à mastocytes.

7. Utilisation selon l'une quelconque des revendications 1 à 6, la latrophiline étant choisie dans le groupe constitué par la latrophiline 1, la latrophiline 2 et la latrophiline 3.

8. Procédé *in vitro* de diagnostic d'un cancer des cellules hématopoïétiques chez un sujet, le procédé consistant à détecter une expression accrue d'une ou de plusieurs isoformes de la latrophiline sur des leucocytes d'un échantillon par rapport à des témoins sains.

9. Procédé selon la revendication 8, un ou plusieurs éléments parmi la latrophiline 1, la latrophiline 2 et la latrophiline 3 étant détectée(s).

10. Procédé selon la revendication 8 ou 9, le procédé consistant en outre à capturer des leucocytes à l'aide d'un agent de capture de latrophiline.

11. Procédé selon la revendication 10, l'agent de capture de latrophiline étant un ligand de la latrophiline ou un anticorps dirigé contre la latrophiline.

12. Procédé selon la revendication 11, l'anticorps dirigé contre la latrophiline étant spécifiquement contre la latrophiline 1, la latrophiline 2 et/ou la latrophiline 3.

13. Procédé selon l'une quelconque des revendications 10 à 12, les leucocytes capturés exprimant la latrophiline étant visualisés.

14. Procédé selon la revendication 13, les leucocytes capturés étant visualisés à l'aide de l'un quelconque des éléments parmi un ligand de la latrophiline ou un anticorps dirigé contre la latrophiline, un anticorps dirigé contre une protéine de surface cellulaire, un marqueur radioactif, un marqueur fluorescent ou une combinaison de ceux-ci.

15. Procédé selon l'une quelconque des revendications 10 à 14, l'agent de capture de latrophiline étant appliqué sur une surface.

16. Procédé selon la revendication 15, la surface revêtue étant une paroi interne d'au moins une cupule d'une plaque de microtitrage.

17. Procédé selon l'une quelconque des revendications 8 à 16, le procédé consistant en outre à améliorer l'expression de la latrophiline sur les leucocytes.

18. Procédé selon la revendication 18, l'expression de la latrophiline étant améliorée à l'aide d'un facteur de croissance et/ou d'une cytokine et/ou d'un agent hématopoïétique et/ou d'un ligand pro-inflammatoire.

19. Procédé selon la revendication 18, la cytokine étant une cytokine pro-inflammatoire ou un agent hématopoïétique.

20. Procédé selon la revendication 18 ou 19, la cytokine ou l'agent hématopoïétique étant un facteur de cellules souches (SCF).

21. Procédé selon la revendication 18, le ligand pro-inflammatoire étant un ligand spécifique au récepteur de type Toll 4 (TLR4).

22. Procédé selon la revendication 21, le ligand spécifique au récepteur de type Toll 4 étant un lipopolysaccharide (LPS).

23. Procédé selon la revendication 22, le lipopolysaccharide étant dérivé de *Pseudomonas aeruginosa.*

24. Procédé selon l'une quelconque des revendications 8 à 23, le procédé consistant en outre à stimuler l'exocytose des leucocytes et à détecter une ou plusieurs protéines, ou des fragments de celles-ci, libérées par les leucocytes par suite de l'exocytose.

25. Procédé selon la revendication 24, l'exocytose étant stimulée par un ou plusieurs ligands de la latrophiline.

26. Procédé selon la revendication 25, le ou les ligands de la latrophiline étant choisis dans le groupe comprenant un anticorps de la latrophiline, une alpha-latrotoxine et une Lasso/téneurine-2.

27. Procédé selon l'une quelconque des revendications 24 à 26, la ou les protéines, ou des fragments de celles-ci, libérées par les leucocytes par suite de l'exocytose étant détectées en liant au moins l'un des éléments parmi un anticorps, un marqueur radioactif, et/ou un ligand fluorescent.

28. Procédé selon l'une quelconque des revendications 24 à 27, la ou les protéines, ou des fragments de celles-ci, libérées par les leucocytes par suite de l'exocytose comportant des cytokines, des hormones et des facteurs de croissance.


### SEQUENCE LISTING

<110> Universitt of Kent, School of Pharmacy
<120> Cancer Diagnostic
<130> UOK-P004WO
<150> GB1510880.6
   <151> 2015-06-19
<160> 8
<170> PatentIn version 3.5
<210> 1
   <211> 23
   <212> DNA
   <213> Artificial
<220>
   <223> Primer sequence for LPHN1
<400> 1
   agccgccccg aggccggaac cta 23
<210> 2
   <211> 29
   <212> DNA
   <213> Artificial
<220>
   <223> Primer sequence for LPHN1
<400> 2
   aggttggccc cgctggcata gagggagtc 29
<210> 3
   <211> 34
   <212> DNA
   <213> Artificial
<220>
   <223> Primer sequence for LPHN2
<400> 3
   cacaacgtcg acctcacact accagtcaag cctg 34
<210> 4
   <211> 35
   <212> DNA
   <213> Artificial
<220>
   <223> Primer sequence for LPHN2
<400> 4
   tggcactatt agagactagt caccagctgc atttg 35
<210> 5
   <211> 24
   <212> DNA
   <213> Artificial
<220>
   <223> Primer sequence for LPHN3
<400> 5
   gacctgggcc tttggactca tgta 24
<210> 6
   <211> 19
   <212> DNA
   <213> Artificial
<220>
   <223> Primer sequence for LPHN3
<400> 6
   cgccgctggc aatgctgta 19
<210> 7
   <211> 17
   <212> DNA
   <213> Artificial
<220>
   <223> Primer sequence for Actin
<400> 7
   ttcgcgggcg acgatgc 17
<210> 8
   <211> 20
   <212> DNA
   <213> Artificial
<220>
   <223> Primer sequence for Actin
<400> 8
   ggggccacac gcagctcatt 20